# EUROPEAN PATENT APPLICATION

(11) **EP 1 574 177 A1**
(43) Date of publication of application: **14.09.2005**
(21) Application number: 05000064.5
(22) Date of filing: 04.01.2005
(51) Int. Cl.: A61B 19/00, A61B 17/15

(54) **Femoral navigation instrument**

(30) Priority: 08.03.2004 US 795621
(71) Applicant: Zimmer Technology, Inc., Illinois 60606 (US)
(72) Inventor: Grimm, James E., Winona Lake Indiana 46590 (US); Patmore, Donald M., Winona Lake Indiana 46590 (US); McGinley, Shawn E., Fort Wayne Indiana 46814 (US); Hall, Maleata Y., Warsaw Indiana 46580 (US); Hui, Sudip, Warsaw Indiana 46582 (US)
(74) Representative: Manitz, Finsterwald & Partner GbR

(57) **Abstract**

A surgical instrument (20) is provided for use with an anatomical structure. The surgical instrument is able to be tracked by a surgical navigation system to guide positioning of the surgical instrument.

## Description

The present invention relates to surgical instruments and, more specifically, to a surgical guide for properly positioning a surgical instrument with respect to an anatomical element.

The controlled positioning of surgical instruments is of significant importance in many surgical procedures and various methods and guide instruments have been developed for properly positioning a surgical instrument. Such methods include the use of surgical guides which function as mechanical guides for aligning drilling, cutting or milling instruments. The use of such surgical guides is common in orthopedic surgical procedures and such guides may be used to properly align a drill or cutting or milling instrument with respect to a bone when preparing the bone for receiving an implant such as an artificial joint. Computer assisted surgical procedures which involve the surgical navigation of a surgical instrument are also known. Surgical navigation techniques typically involve acquiring preoperative images of the relevant anatomical structures and generating a data base which represents a three dimensional model of the anatomical structures. The relevant surgical instruments typically have known and fixed geometries which are also defined preoperatively. During the surgical procedure, the position of the instrument being used is registered with the anatomical coordinate system and a graphical display showing the relative positions of the tool and anatomical structure may be computed in real time and displayed for the surgeon to assist the surgeon in properly positioning and manipulating the surgical instrument with respect to the relevant anatomical structure.

In surgical navigation procedures, a robotic arm may be used to position and control the instrument, or, the surgeon may manually position the instrument and use the display of the relative position of the instrument and anatomical structure when positioning the instrument.

The present invention provides a surgical instrument for use with an anatomical structure. The surgical instrument is able to be tracked by a surgical navigation system to guide positioning of the surgical instrument.

In one aspect of the invention, a surgical instrument for use with an anatomical structure includes an anchoring member having a first portion securable to the anatomical structure and a base body adjustably repositionable relative to the anchoring member. The surgical instrument further includes at least one reference element mountable to the base body and able to be tracked by a surgical navigation system.

In another aspect of the invention, a surgical instrument for use in an orthopaedic surgical procedure on a distal portion of a femur includes a base member, a guide member, and a reference member. The base member includes an anchoring member having a first portion securable to the femur and a base body adjustably repositionable relative to the anchoring member. The guide member is mountable to the base member to establish a datum for guiding a subsequent surgical component. The reference member is mountable to the base member and trackable by a surgical navigation system to determine the position of the guide member relative to the femur.

A method of positioning a surgical instrument with respect to an anatomical structure includes: providing an instrument having a base member comprising an anchoring member and a base body adjustably repositionable relative to the anchoring member; positioning the anchoring member relative to the anatomical structure with the aid of a computer implemented surgical navigation system; securing the anchoring member to the anatomical structure in a selected position; and selectively adjusting the position of the base member relative to the anchoring member with the aid of a computer implemented surgical navigation system after securing the anchoring member to the anatomical structure. Preferably, the anchoring member comprises a threaded shaft and positioning the anchoring member includes engaging the anchoring member with a rotary driver having a surgical navigation system reference member mounted to it and positioning the anchoring member to a predetermined depth relative to the anatomic structure. Preferably, the method further comprises: attaching a guide member to the base member such that adjusting the position of the base member relative to the anchoring member adjusts the position of the guide member relative to the anatomical structure. Preferably, the method comprises: using the guide member to establish a datum to guide a subsequent surgical component. Preferably, the anatomical structure comprises the distal portion of a femur and the guide member comprises a distal femoral cut guide such that adjusting the position of the base member relative to the anchoring member adjusts the varus-valgus and flexion-extension angles of the guide relative to the distal femur. Preferably, the method further comprises: securing the guide member to the femur by inserting fixation members through the guide member. Preferably, the method further comprises: removing the anchoring member, leaving the guide member secured to the femur; and guiding a cutter with the guide member to cut the femur.

Various examples of the present invention will be discussed with reference to the appended drawings. These drawings depict only illustrative examples of the invention and are not to be considered limiting of its scope.

FIG. 1 is an exploded perspective view of a surgical instrument in accordance with the present invention;

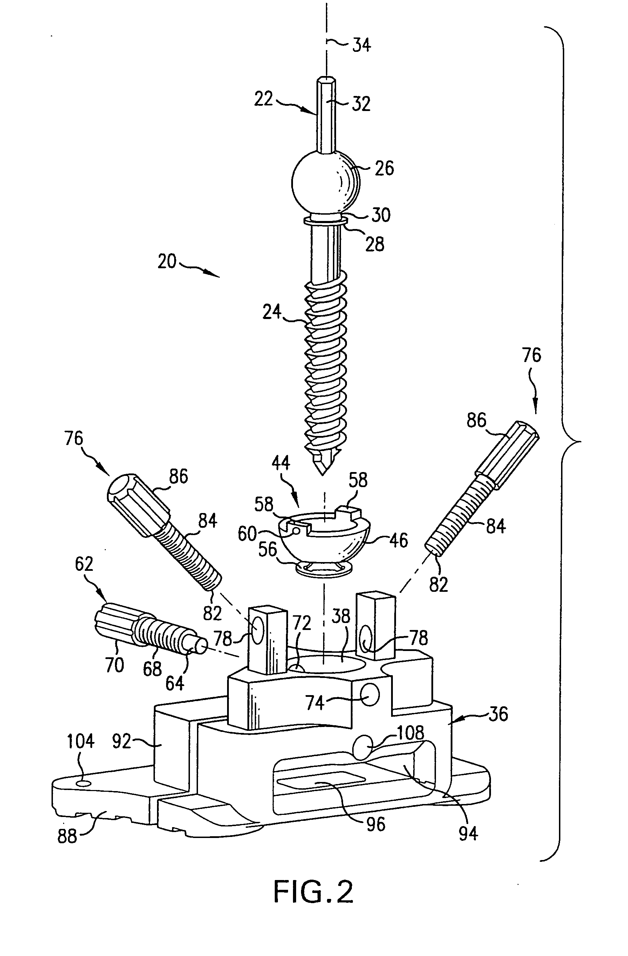

FIG. 2 is another exploded perspective view of the surgical instrument of FIG. 1;

FIG. 3 is a front elevation view of the surgical instrument of FIG. 1;

FIG. 4 is a top plan view of a reference member having reference elements disposed thereon;

FIG. 5 is a side elevation view of the reference member of Figure 4;

FIG. 6 is a front elevation view of a femur and a tibia;

FIG. 7 is a perspective view of a base structure and cutting guide that can be used with the surgical instrument of FIG. 1;

FIG. 8 is an exploded perspective view of an illustrative alternative configuration of the surgical instrument of FIG. 1;

FIG. 9 is a side cross sectional view of the surgical instrument of FIG. 8;

FIG. 10 perspective view of a portion of the surgical instrument of FIG. 8 in use with a driver and a bone; and

FIG. 11 is a perspective view of the surgical instrument of FIG. 8 in use with a bone.

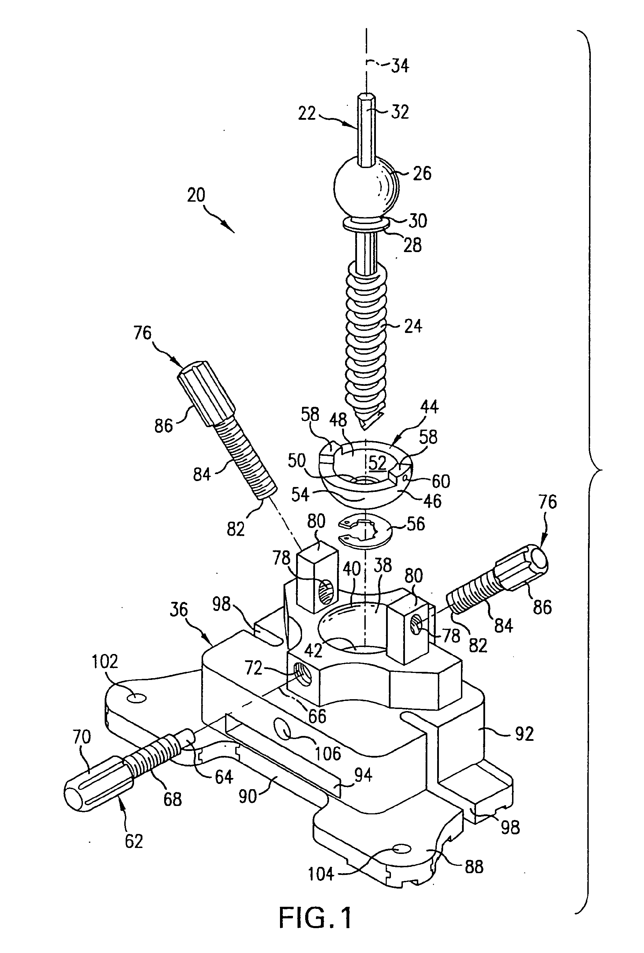

In accordance with the present invention, a surgical instrument 20 is shown in Figure 1. Instrument 20 includes an anchoring member 22. Anchoring member 22 has a first portion formed by threaded shaft 24 which is securable to an anatomical structure such as a bone. Shaft 24 has a configuration similar to the threaded shafts of conventional bone screws. Anchoring member 22 also includes a spherical portion 26. Located between the threads of threaded shaft 24 and spherical portion 26 is a collar 28 which defines an annular recess 30 between collar 28 and spherical portion 26. A hexagonal shaped shaft 32 is located coaxially with threaded shaft 24 on the opposite side of spherical portion 26. Hexagonal shaft 32 is engageable with a rotary driving device to rotate anchoring member 22 about axis 34 defined by shaft 24 and threadingly engage shaft 24 with an anatomical structure.

Anchoring member 24 is adjustably mounted on instrument body 36. Instrument body 36 defines a partially spherical recess 38 having oppositely disposed openings 40, 42. Opening 40 has a larger diameter than opening 42. Pivotal bearing 44 is mounted in recess 38. Bearing 44 includes a partially spherical shell portion 46 with oppositely disposed openings 48 and 50. Opening 48 has a larger diameter than opening 50. Spherical portion 26 of anchoring member 22 is seated within bearing 44 and bearingly contacts inner surface 52 of bearing 44. Outer surface 54 of bearing 44 bearingly contacts the surface of recess 38.

To assemble instrument 20, threaded shaft 24 is inserted through openings 48, 50 of bearing 44 and spherical portion 26 is retained within shell portion 46 by installing retaining clamp 56 in annular recess 30 to prevent shaft 24 from being retracted through opening 50. Bearing 44 includes tabs 58 having openings 60 and is pivotally mounted to instrument body 36 by inserting reduced diameter tips 64 of adjustment members 62 into openings 60. When mounted, bearing 44 pivots about axis 66 defined by adjustment members 62. Adjustment members 62 (only one is shown in the Figures) have a threaded portion 68 and a grip portion 70. Threaded portions 68 are engaged with threaded bores 72 and 74 in instrument body 36. When adjustment members 62 are relatively loosely tightened, bearing 44 is pivotal on tips 64. As one, or both, adjustment members 62 are tightened, bearing 44 becomes firmly engaged between adjustment members 62 and is thereby securable in a selected rotational position relative to axis 66.

Adjustment members 76 are mounted in threaded bores 78 located in projections 80 positioned adjacent recess 38 on instrument body 36. Threaded bores 78 are positioned at an angle relative to projections 80 so that distal ends 82 of adjustment members 76 are engageable with spherical portion 26 of anchoring member 22. Adjustment members 76 also include a threaded shaft 84 and a grip portion 86. Distal ends 82 of members 76 may form a portion of a sphere having the same radius of spherical portion 26 to provide a greater area of contact between distal ends 82 and spherical portion 26. When members 76 are relatively loosely tightened, spherical portion 26 may rotate relative to distal ends 82. Tightening members 76 firmly engages distal ends 82 with spherical portion 26 to secure anchoring member 22 in a selected position relative to instrument body 36.

Instrument body 36 includes a base portion 88 which includes a recess 90. Instrument body 36 also includes a central portion 92. A slot 94 is defined between base portion 88 and central portion 92. An opening 96 allows for the passage of threaded shaft 24 through base portion 88. Also defined by instrument body 36 are opposed slots 98 and bore holes 102, 104, 106 and 108.

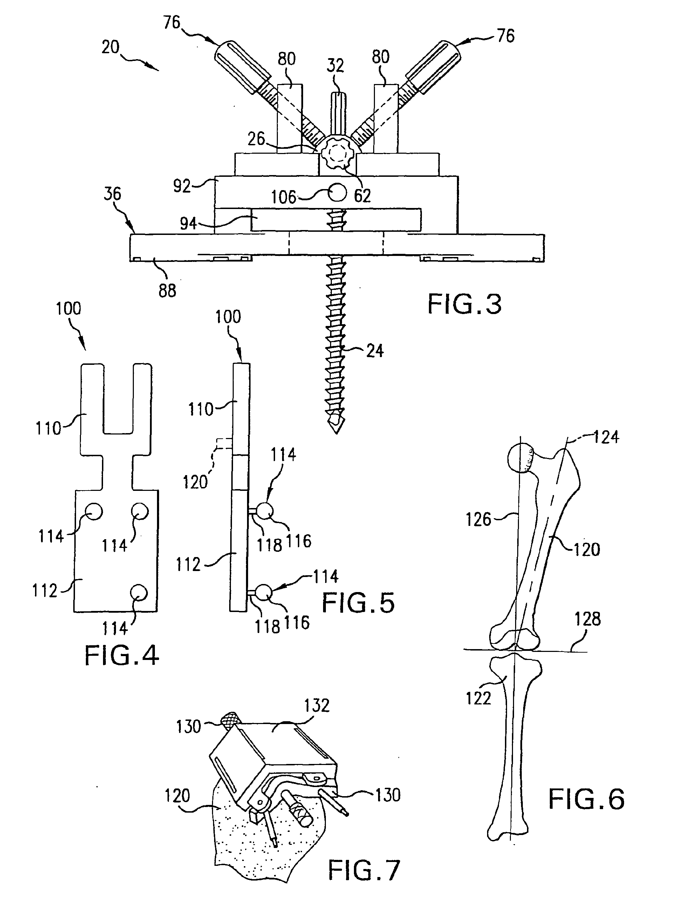

A reference member 100 is shown in Figures 4 and 5. The reference member 100 includes a fork-shaped mounting portion 110 and a registration portion 112. Mounted on the registration portion 112 is a plurality of reference elements 114 which are detectable by a surgical navigation system. The reference elements may be detectable electromagnetically, acoustically, by imaging, or by other suitable detection means. Furthermore, the reference element may be active or passive. Examples of active tracking elements may include electromagnetic field emitters in an electromagnetic system, light emitting diodes in an imaging system, and ultrasonic emitters in an acoustic system, among others. Examples of passive tracking elements may include elements with reflective surfaces. In the disclosed embodiment, three non-linearly positioned reference elements 114 are mounted on reference member 100 and have a spherical portion 116 mounted on a post 118. Spherical portion 116 is a reflective structure which is used to reflect light to facilitate the detection and registration of reference elements 114 in a computer implemented surgical navigation system as discussed in greater detail below.

Reference member 100 is removably mountable to instrument body 36 by positioning mounting portion 110 in slot 94. Mounting portion 110 is configured to closely fit slot 94 so that mounting of reference member 100 will position reference elements 114 at known relative positions and orientations to instrument body 36. Reference member 100 may optionally include a projection 120 extending transverse to the length of forked mounting portion 110 and which fits within recess 90 to facilitate the mounting of reference member 100 at a known and reproducible relative position to instrument body 36.

In alternative embodiments, reference elements 114 may be permanently secured to instrument body 36 or individually removably mounted to instrument body 36 such as to bore holes 102, 104, 106 and/or 108. Alternative reference elements may also include radio-opaque reference elements. If radio-opaque reference elements are employed, reference member 100 may be formed of a radio-transparent material and advantageously position reference elements 114 at a distance from instrument body 36 which, in the illustrated embodiment is formed of stainless steel, a radio-opaque material which could interfere with the detection of radio-opaque reference elements positioned in close proximity to instrument body 36. In the illustrated embodiment, reference member 100 is an aluminum structure. The use of a removably mounted reference member 100 having reference elements 114 mounted thereon facilitates the use of instrument body 36 with different types of surgical navigation systems by allowing different reference members having the same physical shape but with different types of reference elements to be used with a single instrument body design.

The relevant dimensions of instrument 20 and the location of reference elements 114 relative to instrument body 36 when reference member 100 is mounted to instrument body 36 can be determined in advance and this data may be entered into a surgical navigation system. The relevant dimensional data concerning the anatomical structure which is the subject of the surgical procedure may also be entered into the surgical navigation system in advance of the surgical procedure.

As is known in the art, the relevant dimensional data concerning an anatomical structure of interest, e.g., a femur, may be determined using data acquired from images of the anatomical structure to generate a data base representing a model of the anatomical structure. The model of the anatomical structure may be a three dimensional model which is developed by acquiring a series of two dimensional images of the anatomical structure. Alternatively, the model of the anatomical structure may be a set of two dimensional images having known spatial relationships or other data structure which can be used to convey information concerning the three dimensional form of the anatomical structure. The model of the anatomical structure may then be used to generate displays of the anatomical structure from various perspectives for preoperative planning purposes and intraoperative navigational purposes. A variety of technologies which may be employed to generate such a model of an anatomical structure are well known in the art and include computed tomography (CT), magnetic resonance imaging (MRI), positron emission tomography (PET), ultrasound scanning and fluoroscopic imaging technologies.

The model of the anatomical structure obtained by such imaging technologies can be used for the intraoperative guidance of a surgical tool by facilitating the determination and display of the relative position and orientation of the surgical tool with respect to the actual anatomical structure. For example, if the model of the anatomical structure is a set of two dimensional images having known spatial relationships, several such images may be simultaneously displayed during the surgical procedure. By also displaying the position of the tool in the images and displaying images taken from different perspectives, e.g., one image facilitating the display of tool movement along the x and y coordinate axes and another image facilitating the display tool movement along the z axis, the individual images may together represent the movement of the tool in three dimensions.

For reference purposes, a coordinate system defined by the actual anatomical structure which is the subject of interest will be referred to herein as the anatomical coordinate system and a coordinate system defined by the model of the anatomical structure will be referred to as the surgical navigation coordinate system. Data concerning the fixed size and shape of the surgical tool, or of a relevant portion thereof, which will be used in the surgical navigation procedure is also determined pre-operatively to obtain a three dimensional model of the tool or the relevant portions thereof.

Rigid anatomical structures, such as skeletal elements, are well suited for such surgical navigation techniques and individual skeletal elements may be used to define separate coordinate systems. The different rigid structures, e.g., skeletal elements, may be subject to relative movement, for example, the femur and tibia of a patient may be relatively moved during the surgical procedure and separate three dimensional models and coordinate systems may be created for the different skeletal elements. For example, during a knee replacement procedure, a three dimensional model of the tibia defining a first coordinate system may be utilized during the resection of the tibia while a separate coordinate system defined by a three dimension model of the femur is utilized during the resection of the femur.

When conducting surgical navigation techniques, the surgical navigation coordinate system is registered with the anatomical coordinate system and the position of the surgical tool is also registered within the surgical navigation coordinate system. After the registration of both the actual anatomical structure and the surgical tool, the relative position and orientation of the surgical tool may be communicated to the surgeon by displaying together images of the anatomical structure and tool based upon the three dimensional models of the anatomical structure and tool which were previously acquired.

Computer implemented surgical navigation systems which provide for the registration of an actual anatomical structure with a three dimensional model representing that structure together with the registration or localization of a surgical tool within the surgical navigation coordinate system to facilitate the display of the relative positions of the surgical tool and the actual anatomical structure are known in the art. Known methods of registering the anatomical structure with the surgical navigation coordinate system include the use of implanted fiducial markers which are recognizable by one or more scanning technologies. Alternatively, implants which may be located by physically positioning a digitizing probe or similar device in contact or at a known orientation with respect to the implant. Instead of using implants, it may also be possible to register the two coordinate systems by aligning anatomical landmark features.

Tracking devices employing various technologies enabling the registration or localization of a surgical tool and the tracking of the tool motion with respect to the anatomical coordinate system, which has been registered with the surgical navigation coordinate system, are also known. For example, optical tracking systems which detect light from reflected or emitted by reflective targets or localizing emitters secured in a known orientation to the tool are known for determining the position of a surgical tool and registering the position of the tool within an surgical navigation coordinate system representing a three dimensional model of an anatomical structure. For example, such a tracking system may take the form of a sensor unit having one or more lenses each focusing on separate charge coupled device (CCD) sensitive to infrared light. The sensor unit detects infrared light emitted by three or more non-linearly positioned light emitting diodes (LEDs) secured relative to the tool. A processor analyzes the images captured by the sensor unit and calculates the position and orientation of the tool. By registering the position of the sensing unit within the surgical navigation coordinate system, the position of the tool relative to the anatomical structure, which has also been registered with the surgical navigation coordinate system, may be determined and tracked as the tool is moved relative to the anatomical structure.

Alternative localizing systems may employ localizing emitters which emit an electromagnetic signal in the radio frequency or which emit visible light. It is also possible to employ digitizing physical probes which are brought into physical contact with the tool at predefined locations on the tool to register the position of the tool.

In the disclosed embodiment, the localizing system includes a light source and reference elements 114 reflect the light. The localizing system then detects the reflected light and computes the location of the individual reference elements 114 in a known manner. Reference elements 114 may be obtained from Northern Digital Inc. having a place of business at 103 Randall Dr., Waterloo, Onterio, Canada, N2V1C5. Other types of localizing systems may also be used with the present invention, such as those employing reflecting elements which emit a signal or which are radio-opaque. Known localizing systems of computer implemented surgical navigation systems may also be used to determine the relative position of a radio-opaque structure having an identifiable shape such as threaded shaft 24. Northern Digital Inc. supplies surgical navigation systems under the brand names Optotrak® and Polaris® which may be used with the present invention.

The use of instrument 20 in the resection of a distal femur will now be discussed. When implanting a prosthetic knee joint, the distal femur must be prepared to receive the femoral implant. The preparation of the distal femur typically involves resecting the distal femur to form several intersecting planar surfaces which conform to the interior surface of the selected femoral component. Figure 6 illustrates a femur 120 and tibia 122. The anatomical axis 124 of femur 120 is defined by the intramedullary canal of femur 120. The mechanical axis 126 of femur 120 extends from the center of the femoral head on the proximal femur to the center of the intercondylar notch on the distal femur. For many individuals the angle between the anatomical axis 124 and the mechanical axis 126 is approximately six degrees.

It is common to use the intramedullary canal of the femur as a reference structure when positioning a resection guide, such as a cutting or milling guide, on the distal femur to properly guide the milling or cutting instrumentation used to resect the distal femur. It is the position of the mechanical axis of the femur, however, which determines the best location of the resection planes to be formed on the distal femur. Thus, when using the intramedullary canal as a reference structure, the difference between the mechanical and anatomical axes of the femur must be addressed. Anchoring member 22 of the present invention, however, can be secured to femur 120 substantially coaxially with the mechanical axis 126 of femur 120.

When securing surgical instrument 20 to femur 120, reference member 100 is mounted to instrument body 36 and registered in the computer implemented surgical navigation system as described above. Similarly, femur 120 is registered within the surgical navigation system. The computer implemented surgical navigation system is then used to position anchoring member 22 coaxially with the mechanical axis of the femur. The location of the mechanical axis is determined preoperatively. For example, the surgeon may input the mechanical axis location into the surgical navigation system by indicating the location of two points on the mechanical axis on the images of the femur. It would also be possible for the surgical navigation system to automatically determine the location of the mechanical axis using the model data representing the femur. Once placed in the selected position coaxially with the mechanical axis, anchoring member 22 is secured to femur 120. When positioning anchoring member 22 relative to femur 120, anchoring member 22 may be placed in a default position relative to body 36 and reference elements 114 mounted on instrument body 36 used to track the position of anchoring member 22 relative to femur 120. Alternatively, anchoring member 22 may be directly detected and tracked by the surgical navigation system.

After anchoring member 22 has been secured to femur 120, the surgical navigation system is used to determine if instrument body 36 is in the desired position relative to femur 120. The desired position of instrument body 36 is determined preoperatively and input into the surgical navigation system. Instrument body 36 is then adjusted relative to anchoring member 22 and femur 120 to align instrument 36 with its desired position. First, instrument body 36 is pivoted about axis 66 of pivotal bearing 44 to obtain the desired varus/valgus alignment, e.g., parallel to the transverse axis 128. When instrument body 36 is in the desired varus/valgus orientation, adjustment members 62 are tightened to prevent pivotal motion of bearing 44. Next, the desired "external rotation" of the instrument body 36 is checked using the surgical navigation system and adjusted if necessary. The external rotation of instrument body 36 refers to the rotational orientation of instrument body 36 relative to axis 34 defined by anchoring member 22 which is positioned coaxially with the mechanical axis 126 of femur 120. After positioning instrument body 36 in the desired rotational position relative to axis 34, adjustment members 76 are firmly engaged with spherical portion 26 to secure instrument body 36 in the desired position relative to anchoring member 22. Anchoring member 24 is then rotated into, or out of, femur 120 to set the "depth" of the resection on the distal femur.

Instrument body 36 is thus adjustable with respect to three degrees of freedom after securing anchoring member 22 with a femur, i.e., instrument body 36 may be rotated about axis 66 and secured in a selected rotational position about axis 66 by tightly engaging adjustment members 62 with pivotal bearing 44; instrument body 36 may be rotated about axis 34 and secured in a selected rotational position about axis 34, which is substantially perpendicular to axis 66, by tightly engaging adjustment members 76 with spherical portion 26; and after initially securing anchoring member 22 to femur 120, instrument body 36 may be translated along axis 34 and placed in a selected position along axis 34 by rotating a translational adjustment member, i.e., anchoring member 22, further into, or out of, femur 120. Although the illustrated embodiment utilizes two adjustment members 62 to positively secure instrument body 36 in a selected rotational position about axis 66 and two adjustment members 76 to positively secure instrument body 36 in a selected rotational position about axis 34, alternative embodiments could employ two individual adjustment members to independently and positively secure instrument body 36 in selected rotational positions about axes 66 and 34.

With regard to the remaining three degrees of freedom, by maintaining relatively tight tolerances between anchoring member 22 and its interfaces with shell portion 46 and opening 50 in bearing 44 and clamp 56, anchoring member 22 can be prevented from pivoting about an axis which is substantially perpendicular to both axes 34 and 66. Rotation about this third axis and the translational position of instrument body 36 along this third axis and axis 66 are all determined by the position and orientation at which anchoring member 22 is engaged with the anatomical structure, e.g., femur 120. Alternative embodiments of the invention allowing the selective adjustment of instrument body 36 relative to anchoring member 22 along one or more of these three remaining degrees of freedom are also possible. The six degrees of freedom referred to herein are defined by translational movement about three substantially mutually perpendicular translational axes and rotational movement about three substantially mutually perpendicular rotational axes and thereby define translational coordinate system and a rotational coordinate system. The translational and rotational axes may be parallel or coincide, however, it is not necessary for such axes to be parallel or coincide.

After being positioned in the desired orientation on the distal femur, instrument body 36 may be used to position base structures 130 on the lateral and medial sides of the distal femur. Surgical instrument 20 may then be removed from the distal femur, a cutting guide 132 secured to base structures 130 as shown in Figure 7 and the distal femur resected with a cutting blade inserted through the various cutting slots defined by cutting guide 132. Femoral bases and cutting guides which may be used with surgical instrument 20 are available under the name 5-in-1 from Zimmer Inc. of Warsaw, Indiana and are described in U.S. Pat. No. 5,743,915 which is hereby incorporated herein by reference. Base structures 130 are positioned on the distal femur by placing base structures 130 into registering contact with instrument body 36 and then securing base structures 130 directly to femur 120. Recess 90, slot 94, openings 102, 104, 106 or 108, slots 98 or other predefined surfaces on instrument body 36 may be used to register a base structure to properly position the base structure on the femur. Alternatively, an intermediate part may be removeably secured to instrument body 36, such as by insertion into a slot or opening on instrument body 36 and the base structure registered with the intermediate part. A cutting or milling guide or other surgical implement could also be formed directly on instrument body 36. Milling and cutting instrumentation which could be adapted for use with an instrument body 36 is disclosed in U.S. Pat. Nos. 5,474,559 and 5,593,411 which are both hereby expressly incorporated herein by reference.

When implanting a prosthetic knee joint using instrument 20, a selectively adjustable surgical instrument that may be used to resect the tibia is described by James E. Grimm in a U.S. Patent Application entitled Surgical Instrument And Positioning Method having an attorney docket number of ZIM0164 and filed on the same date as the present application and is expressly incorporated herein by reference.

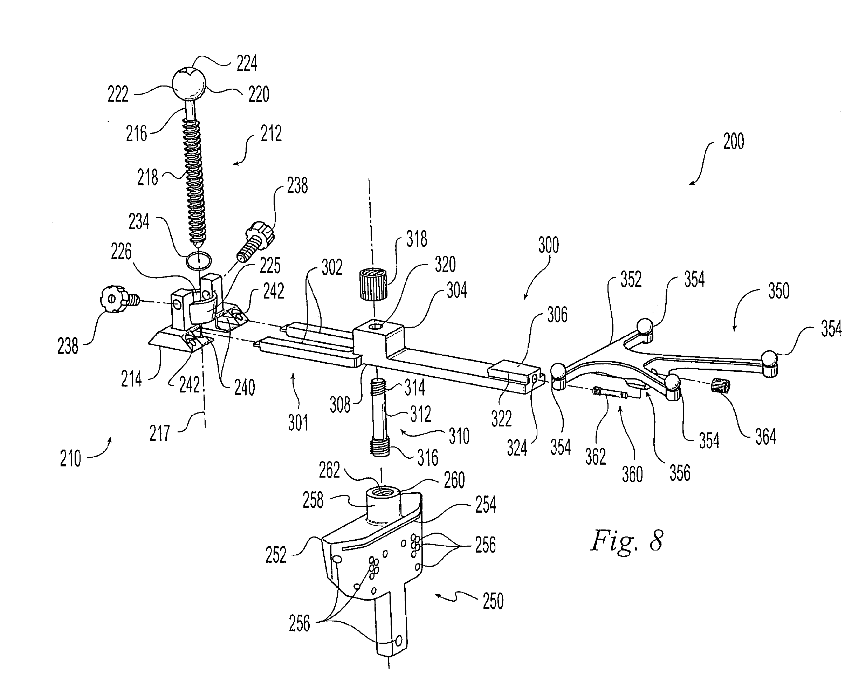

FIGS. 8-11 depict an alternative illustrative arrangement for the surgical instrument of FIG. 1. The instrument 200 includes a base member 210 for mounting on a bone, a guide member 250 for establishing a datum to guide another surgical component, a connecting link 300 for connecting the base member 210 to the guide member 250, and a reference member 350 to facilitate tracking the instrument 200 with a surgical navigation system to position the guide member 250 in a desired position as indicated by the surgical navigation system.

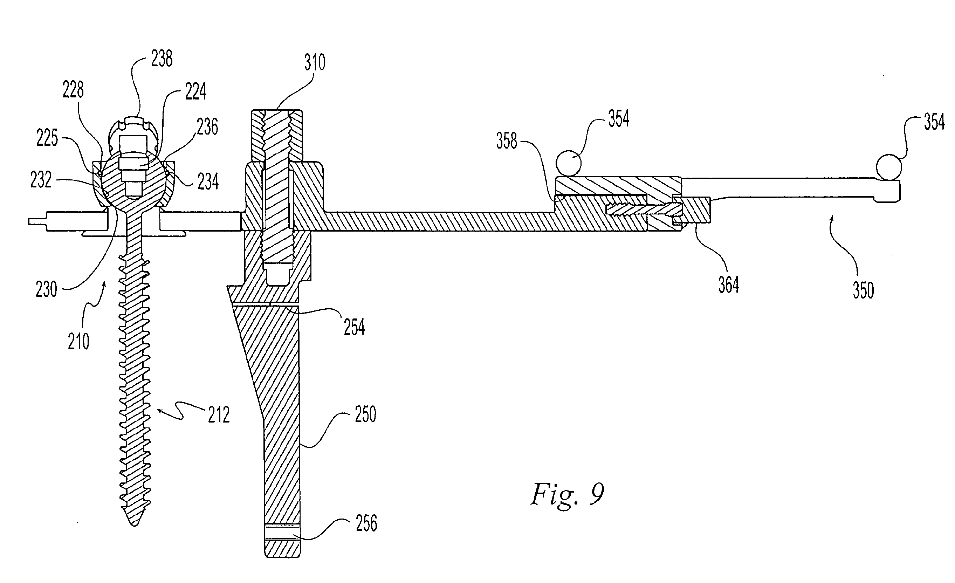

The base member 210 includes an anchoring member 212 and a base body 214 mounted on the anchoring member 212. The anchoring member 212 includes an elongated shaft 216 having a shaft axis 217, threads 218 for engaging a bone at one end of the shaft, and a head 220 at an opposite end of the shaft for engaging the base body 214. The illustrative head 220 includes a spherical portion 222 to facilitate angular adjustment in multiple planes of the base body 214 relative to the anchoring member axis 217. The head 220 also includes a driver portion 224 for engaging a driver to rotate the anchoring member 212. The illustrative driver portion 224 includes a square recess to engage a square driver. However, other driver portion 224 shapes are contemplated and are considered within the scope of the invention. For example, the driver portion may be a female recess or a male projection and it may have any cross sectional shape that will allow it to engage a driver in torque transmitting relationship. The base body 214 includes a bearing cup 225 having an at least partially spherical recess 226 having a top opening 228 and a bottom opening 230. The top opening 228 is larger than the diameter of the spherical portion 222 of the anchoring member 212 to permit the spherical portion 222 to enter the recess 226 through the top opening 228. The bottom opening 230 is smaller than the diameter of the spherical portion 222 of the anchoring member 212 to keep the spherical portion 222 from passing through the recess and to provide a bearing surface 232 for the spherical portion 222. A retention ring 234 fits in an annular groove 236 (FIG. 9) formed in the wall of the recess 226 to retain the anchoring member 212 in the recess 226. The retention ring 234 is positioned between top opening 228 and the center of the spherical portion 222 of the anchoring member 212 to resist movement of the anchoring member 212 toward the top opening 228. The retention ring 234 may be sized to allow the anchoring member 212 to be snapped in and out of the recess 226 or it may be sized such that it is inserted after the anchoring member 212 to retain the anchoring member permanently. With the anchoring member 212 seated in the recess 226, the base body 214 may be pivoted about the anchoring member shaft axis 217 and angled relative to the anchoring member shaft axis 217 in multiple planes passing through the axis 217.

The instrument 200 includes a locking mechanism for locking the base body 214 in a desired position relative to the shaft axis 217. The illustrative instrument 200 includes locking screws 238 threaded into the base body 214 and directed toward the head 220 of the anchoring member 212 so that the screws 238 may be tightened against the head 220 to lock the relative position of the base body 214 and the anchoring member 212.

The instrument 200 includes a mechanism for connecting the base body 214 to the connecting link 300. The illustrative instrument 200 includes connector openings 240 formed in the base body 214 for receiving a portion of the connecting link 300. The base body 214 also includes a supplemental bone anchoring mechanism in the form of fixation holes 242 for receiving fixation members such as screws, pins, nails, and/or other suitable fixation members.

The illustrative connecting link 300 includes a base member connecting portion 301, a guide member connecting portion 304, and a reference member connecting portion 306. The illustrative base member connecting portion 301 includes connecting rods 302 that insert into the connector openings 240 in the base body 214. The illustrative connecting rods 302 and connector openings 240 have rectangular cross sections, but other cross sectional shapes may be used. The illustrative guide member connecting portion 304 includes an abutment surface 308 for abutting a portion of the guide member 250 and a guide member connecting screw 310 for drawing the guide member 250 against the abutment surface 308. The screw 310 may be a two-piece assembly having a shaft 312 threaded at first and second ends 314, 316 and a knob 318 threadably engageable with the first end 314 of the shaft 312. The first end 314 may have a smaller diameter than the second end 316 to allow the first end to pass through a bore 320 in the guide member connecting portion 304 of the connecting link 300 while the second end 316 is prevented from passing through the bore 320. After the first end 314 is passed through the bore 320, the knob 318 may be threaded onto the first end 314 thereby trapping the screw 310 on the connecting link 300. The illustrative reference member connecting portion includes a dovetail mount 322 and a threaded bore 324 for receiving a locking screw.

The guide member 250 includes a mechanism for establishing a datum relative to a bone such as one or more pins, screws, bars, fins, rails, dovetails, planar surfaces, holes, slots, notches, and/or any other suitable datum in or on a bone. The datum may be used to reference the position and/or orientation of a subsequent surgical component including cutting instruments, reaming instruments, templates, drill guides, provisional implants, implants, and/or other components for any suitable surgical site. Examples of surgical sites include hip joints, knee joints, vertebral joints, shoulder joints, elbow joints, ankle joints, digital joints of the hand and feet, fracture sites, tumor sites, and/or other suitable orthopaedic surgical sites. The guide member 250 may be used to establish datums that may be referenced by components that are not otherwise usable with a surgical navigation system. Thus, the guide member 250 may be used to provide the benefits of three dimensional surgical navigation technology while using existing non-navigated components. The guide member 250 may serve as the datum itself to engage and guide a subsequent surgical component directly, or it may be configured to establish a separate intermediate datum A guide member 250 that serves directly as the datum may include one or more pins, screws, bars, fins, rails, dovetails, planar surfaces, holes, slots, notches, and/or other feature that directly engages the subsequent component to guide it relative to a surgical site. For example, the illustrative guide member 250 includes a body 252 having a slot 254 to receive and guide a cutter to produce a cut surface on a bone. The guide member body 252 also includes holes 256 that may receive fixation members to anchor the body 252 relative to the bone while the cutter is in use. Alternatively, the slot 254 and/or holes 256 may be used to establish a separate intermediate datum. For example, the guide member 250 may be used to guide insertion of pins into the bone that are left in place and engaged by a subsequent cut guide to position the cut guide on the bone.

The guide member 250 includes a mechanism for connecting to the connecting link 300. The illustrative guide member 250 includes a boss 258 having an abutment surface 260 and a threaded bore 262. The threaded bore 262 receives the guide member connecting screw 310 such that tightening of the screw 310 draws the guide member abutment surface 260 into engagement with the connecting link abutment surface 308. With the screw 310 securely tightened, the guide member 250 is positioned in predetermined known relationship to the connecting link 300.

The reference member 350 includes a reference member body 352 supporting reference elements 354. The reference member 350 includes a connecting link connection portion 356. The illustrative connection portion 356 includes a dovetail opening 358 (FIG. 9) engageable with the connecting link dovetail 306 and a screw 360 for locking the reference member 350 on the connecting link 300. The screw 360 comprises a two piece assembly having a shaft 362 and knob 364 and is trapped on the reference member 350 in a way similar to the way the guide member connecting screw 310 is trapped on the connecting link 300.

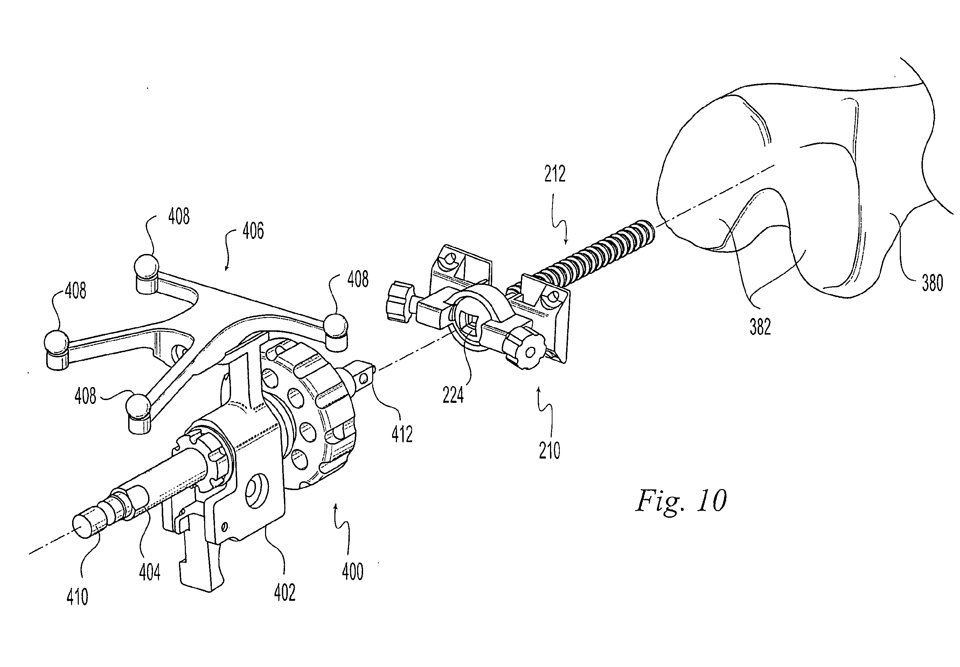

In use, the base member 210 is assembled with the anchoring member 212 inserted into the recess 226. The anchoring member 212 is then screwed into a bone at the surgical site. For example, in a knee replacement surgical procedure, the anchoring member 212 may be screwed into the femur 380 as shown in FIG. 10. The depth of surgical instrument 200 relative to the femur 380 is set by the insertion depth of the anchoring member 212. The angular position of surgical instrument 200 relative to the femur 380 is set by angling the base body 214 relative to the anchoring member 212. To permit maximum angular adjustability of the base body 214, it is desirable to insert the anchoring member 212 generally perpendicular to the femoral condyles 382 on the end of the femur 380 along the mechanical axis of the femur 380.

A navigated driver 400 may be used to facilitate insertion of the anchoring member 212 in the desired location. The driver 400 includes a body 402, a shaft 404 mounted for rotation within the body 402, and a driver reference member 406 having reference elements 408 trackable by the surgical navigation system. One end 410 of the shaft 404 may be configured to connect to a rotary handpiece or handle to provide rotary input to the shaft 404. Another end 412 of the shaft 404 is configured to engage the anchoring member driver portion 224. The illustrative engagement end 412 of the shaft 404 includes a square cross section to engage a square recess defining the anchoring member driver portion 224. With the end of the shaft 412 engaging the anchoring member driver portion 224, the position of the anchoring member 212 relative to the femur may be determined by the surgical navigation system. Under the guidance of the surgical navigation system, the driver 400 may be used to drive the anchoring member 212 along the mechanical axis of the femur 380, or along any other desired path, and to a desired depth relative to the femur 380. Once the anchoring member 212 is positioned, the driver 400 may be removed.

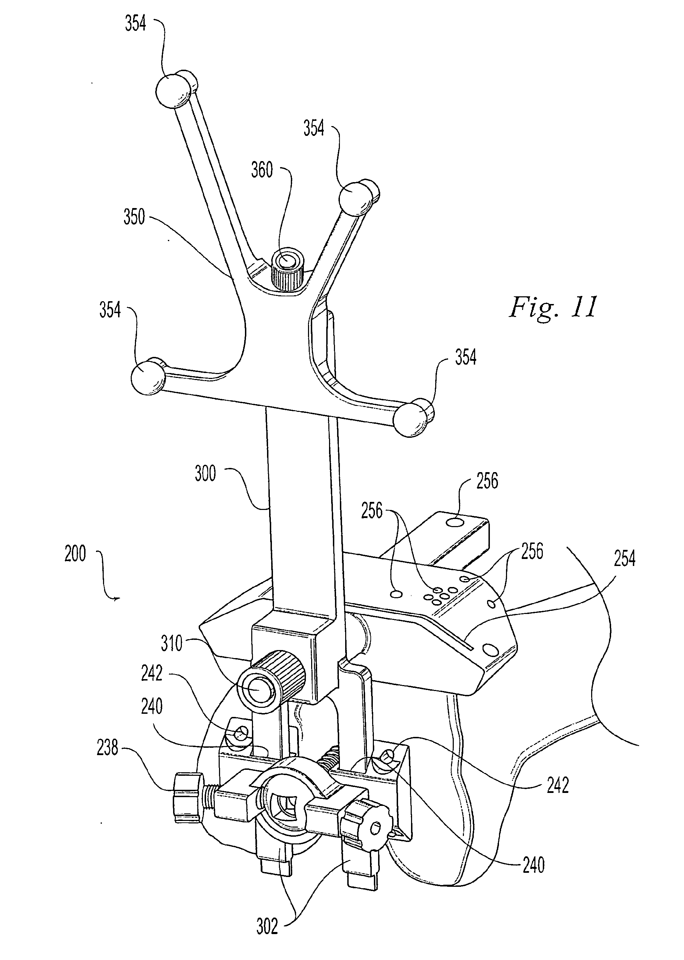

The connecting link 300 is attached to the base member 210 by inserting the connecting rods 302 into connector openings 240 as shown in FIG. 11. The reference member 350 is attached to the connecting link 300 by mating the dovetail 322 of the connecting link 300 with the dovetail opening 358 of the reference member 350 and securing it with the screw 360. The guide member 250 is attached to the connecting link by threading the guide member connecting screw 310 into the threaded bore 262 of the boss 258 until the abutment surfaces 260, 308 are pressed together. The angle of the guide member 250 relative to the femur 380 is set by tilting and pivoting the base member 210 relative to the anchoring member 212 under the guidance of the surgical navigation system. In the illustrative example, the guide member 250 is shown in use to guide the distal femoral cut in knee replacement surgery. In this application, the angular adjustment establishes the flexion angle and the varus-valgus angle of the guide member 250. Once the desired angles are achieved, as indicated by the surgical navigation system, the position is locked by tightening the lock screws 238. The angles may be further secured by inserting fixation members through the fixation holes 242 in the base body 214. Alternatively, the angle of the base member 210 may be adjusted and locked prior to attaching the guide member 250 to the connecting link 300.

The guide member 250 may be used to establish a datum on the femur 380 that is referenced by a subsequent surgical component. For example, one or more pins may be inserted through one or more of the holes 256 in the guide member 250 and a cut guide subsequently engaged with the pins. Alternatively, the guide member 250 may itself serve as the datum by directly guiding a subsequent surgical instrument. For example, a cutter may be inserted through the slot 254 to cut the distal femur. Fixation members may be inserted through one or more of the holes 256 to stabilize the guide member 250. The connecting link 300, reference member 350, and base member 210 may be removed prior to cutting the femur.

While this invention has been described as having an exemplary design, the present invention may be further modified within the spirit and scope of this disclosure. This application is therefore intended to cover any variations, uses, or adaptations of the invention using its general principles.

## Claims

1. A surgical instrument for use with an anatomical structure, the instrument comprising:
an anchoring member having a first portion securable to the anatomical structure;
a base adjustably repositionable relative to the anchoring member; and
at least one reference element mountable to the base , the at least one reference element being able to be tracked by a surgical navigation system.

2. The surgical instrument of claim 1 wherein the anchoring member has a longitudinal axis and the base is repositionable relative to the anchoring member by angling the base in a plurality of planes passing through the anchoring member longitudinal axis.

3. The surgical instrument of claim 2 wherein the anchoring member includes a first portion that is at least partially spherical and the base includes a second portion that is at least partially spherical, the second portion engaging the first portion in spherical bearing engagement such that the second portion may be pivoted about the longitudinal axis and angled in a plurality of planes passing through the longitudinal axis.

4. The surgical instrument of anyone of the preceding claims further comprising a locking mechanism for locking the position of the base relative to the anchoring member.

5. The surgical instrument of claim 3 or 4 wherein one of the first and second portions comprises an at least partially spherical concave portion and the other of the first and second portions comprises an at least partially spherical convex portion received within the concave portion, the concave portion including an annular groove and a retaining ring disposed in the annular groove to retain the convex portion within the concave portion.

6. The surgical instrument of anyone of the preceding claims further comprising a guide member mountable to the base to establish a datum for guiding a subsequent surgical component.

7. The surgical instrument of claim 6 wherein the position of the guide member relative to the anatomical structure may be determined by the surgical navigation system by tracking the at least one reference element.

8. The surgical instrument of claim 6 or 7 wherein the guide member is engageable by a cutter to guide the cutter to cut a portion of the anatomical structure.

9. The surgical instrument of anyone of claims 6 to 8 wherein the guide member is engageable by an elongate datum member to guide the elongate datum member to a desired position relative to the anatomical structure.

10. The surgical instrument of anyone of claims 6 to 9 wherein the anatomical structure comprises a distal portion of a femur adjacent a knee joint and the base is adjustably repositionable relative to the anchoring member to adjust a varus-valgus angle and a flexion-extension angle of the guide member relative to the distal portion of the femur.

11. The surgical instrument of claim 10 wherein the anchoring member includes a threaded shaft threadably engageable with the femur and the guide member is engageable by a cutter to guide the cutter to cut the distal portion of the femur.

12. A surgical instrument for use in an orthopaedic surgical procedure on a distal portion of a femur, the instrument comprising:
a base member including an anchoring member having a first portion securable to the femur and a base body adjustably repositionable relative to the anchoring member;
a guide member mountable to the base member to establish a datum for guiding a subsequent surgical component; and
a reference member mountable to the base member and trackable by a surgical navigation system to determine the position of the guide member relative to the femur.

13. The surgical instrument of claim 12 wherein the anchoring member includes a first portion that is at least partially spherical and the base body includes a second portion that is at least partially spherical, the second portion engaging the first portion in spherical bearing engagement such that the second portion may be pivoted about the longitudinal axis and angled in a plurality of planes passing through the longitudinal axis to adjust a varus-valgus angle and a flexion-extension angle of the guide member relative to the distal portion of the femur.

14. The surgical instrument of claim 12 or 13 further comprising a connecting link mountable to the base member, the guide member and the reference member being mountable to the connecting link to mount them to the base member.

15. The surgical instrument of claim 14 wherein the base body includes connector openings and the connecting link includes connector rods engageable with the connector openings to mount the connecting link to the base body.

16. The surgical instrument of anyone of claims 12 to 15 wherein the guide member comprises a distal femoral cut guide able to guide a cutter to cut the distal portion of the femur.

17. The surgical instrument of anyone of claims 14 to 16 wherein the connecting link comprises an elongate body having a first end, a second end, and an intermediate portion between the first and second ends, the base body connecting to the connecting link at the first end, the reference member connecting to the connecting link at the second end, and the guide member connecting to the connecting link at the intermediate portion.
